# EUROPEAN PATENT SPECIFICATION

(11) **EP 1 026 505 B1**
(45) Date of publication and mention of the grant of the patent: **02.10.2002**
(21) Application number: 99116336.1
(22) Date of filing: 19.08.1999
(51) Int. Cl.: G01N 33/68

(54) **A complex buffer system for the CZE analysis of hirudin**
Komplexes Puffersystem zur CZE-Analyse von Hirudin
Système de tampon complexe pour l'analyse CZE de l'hirudine

(43) Date of publication of application: 09.08.2000
(73) Proprietor: SCHERING AKTIENGESELLSCHAFT, 13342 Berlin (DE)
(72) Inventor: Dönges, Reiner, 35041 Marburg (DE); Brazel, Dieter, Dr., 35041 Marburg/Lahn (DE); Aretz, Waltraud, Dr., 60320 Frankfurt am Main (DE)

(56) References cited:
- ODA, R. P.; MADDEN, B. J.; SPELSBERG, T. C.; LANDERS, J. P.: ".alpha.,.omega.-Bis-quaternary ammonium alkanes as effective buffer additives for enhanced capillary electrophoretic separation of glycoproteins" J. CHROMATOGR., A, vol. 680, no. 1, 1994, pages 85-92, XP000861577
- ODA, R. P.; MADDEN, B. J.; MORRIS, J. C.; SPELSBERG, T. C.; LANDERS, J. P. : "Multiple-buffer-additive strategies for enhanced capillary-electrophoretic separation of peptides" J. CHROMATOGR. A, vol. 680, no. 1, 1994, pages 341-351, XP000861578
- DETTE, C.; WAETZIG, H.: "Separation of r-hirudin from similar substances by capillary electrophoresis " J. CHROMATOGR., A, vol. 700, no. 1-2, 1995, pages 89-94, XP004023413
- HORTIN, GLEN L.; GRIEST, TERRY; BENUTTO, BARBARA M.: "Separations of sulfated and nonsulfated forms of peptides by capillary electrophoresis: comparison with reversed-phase HPLC" BIOCHROMATOGRAPHY, vol. 5, no. 3, 1990, pages 118-120, XP000870275
- LUEDI, H.; GASSMANN, E.; GROSSENBACHER, H.; MAERKI, W.: "Capillary zone electrophoresis for the analysis of peptides synthesized by recombinant DNA technology" ANAL. CHIM. ACTA, vol. 213, no. 1-2, 1988, pages 215-219, XP000869524
- M M BUSHEY, J W JORGENSON: "Capillary electrophoresis of proteins in buffers containing high concentraions of zwitterionic salts" JOURNAL OF CHROMATOGRAPHY, vol. 480, 1989, pages 301-310, XP000263867
- R P ODA, J P LANDERS: "Effect of cationic buffer additives on the capillary electrophoretic separation of serum transferrin from different species" ELECTROPHORESIS, vol. 17, 1996, pages 431-437, XP000866498

## Description

The present invention concerns an analytical method for the precise and reproducable determination of the structural deviants of biotechnological produced hirudin by means of capillary electrophoresis.

Since although the well established RP-HPLC seems to be the analytical method of choice, in the case of recombinant hirudin less adequate baseline separation of the structural deviants is achieved. Overall HPLC alone is no longer sufficient to provide characterization information for biotechnology derived proteins.

Capillary electrophoresis (CE) is one of the new technologies that is being used as a complementary technique in this process. Together, HPLC and CE can accomplish a separation that is difficult by either technique alone, especially to provide evidence of batch-to-batch consistency of production, as well as performing in process control and stability studies. In the same way, CE can play an important role in the complete characterization and, therefore, validation of biotechnology drugs.

In addition to the many applications for HPCE protein analysis that have been published during the last few years, new ones are necessary and must be developed.

In capillary electrophoresis, molecules are separated based on their differences in electrical charge and structure. Their molecular migration rate depends on their electrophoretic mobility (the movement of the charged particles towards the opposite charged electrode) and the speed of the electroendosmotic flow (the flow rate of the electrolyte solution which, due to charges on the inner surface of the capillary, is induced when an electrical field is applied).

First of all, the composition of the buffer is the key element contributing to the success of a CE separation. The type of buffer, its pH, ionic strength and additives as well as viscosity, and last but not least the properties of the capillary wall affect the efficiency, selectivity, and resolution of the separation.

The polypeptide hirudin is a potent and specific thrombin inhibitor for anticoagulant therapy. In conclusion of the establishement of gene-technology methods, nowadays recombinant hirudin can be produced, but without the avoidance of minor impurities.

Besides of RP-HPLC, Capillary Electrophoresis (HPCE) seemes to be a versatile tool to separate these closely related proteins from the main product, because although the structural differences of r-hirudin and its degradation products are of a minor aspect, purity evaluation is essential whenever synthesized polypeptides like recombinant hirudin are used therapeutically.

As a means of evaluating the potential for developing a potency capillary electrophoresis - based assay for the analysis of r-hirudin, the capillary zone electrophoretic (CZE) analysis of r-hirudin from several similar species was evaluated using an uncoated fused silica capillary and a separation augmented with buffer additives.

### Hirudin

The polypeptide hirudin, which was originally isolated form the leech Hirudo medicinalis, is a highly specific thrombin inhibitor possessing a broad therapeutic potential. However, it is only possible to prepare the quantities which are required by the recombinant route using transformed microorganisms. In this context, it has been found that the yeast Saccharomyces cerevisiae is suitable as a host organism for producing hirudin which is correctly folded and fully active (EP-A1 0 168 342, EP-A1 0 200 655).

Hirudin is to be understood to mean peptide-like thrombin inhibitors which have a specific activity of at least 10,000 ATU/mg (anti-thrombin units/mg), which are derived from the known isohirudins of the species Hirudo medicinalis and which exhibit essential structural features of these isohirudins, in particular the characteristic linking of the three disulfide bridges, (cf., for example, EP-A1 0 158 564, EP-A1 0 168 342, DE 34 45 517, EP-A2 0 193 175, EP-A1 0 200 655, EP-A1 0 158 986, EP-A1 0 209 061, DE 33 42 199, EP-A 0 171 024).

In particular, among these, a hirudin is understood to be a hirudin as described in EP-A1 0 171 024, EP-A1 0 158 986 and EP-A1 0 209 061.

During the past several years a renewed interest in the application of hirudin as a therapeutic drug has become evident [1]. Hirudin from the leech Hirudo medicinalis is the most potent and most specific known inhibitor of thrombin, the serine protease that functions in regulating hemostasis and that catalyzes the final steps in blood coagulation - the conversion of fibrinogen to clottable fibrin [2].

Hirudin is now available in large amounts as it is produced by genetic engineering. As a production host Saccharomyces cerevisiae is used. One particular advantage of this host is the possibility of harnessing the secretory apparatus of the cell to express hirudin and transport it directly into the surrounding culture medium. This mode of production can facilitate processing and should result in the synthesis of homogeneous correctly processed material. Stable inheritance is most commonly achieved by using an auxotrophic host strain with the plasmid providing the complementing function. For the isolation, concentration of the crude clarified supernatant is achieved using low molecular weight UF-membranes and conventional ion-exchange chromatography [3]. Purification of the final product is accomplished using ion-exchange and RP-HPLC steps.

In contrast to the natural molecule which contains a sulfated tyrosine residue in position 63, recombinant hirudin produced in Saccharomyces cerevisiae is not sulfated [4].

The sequence of the yeast hirudin has been determined by automated Edman degradation. It is 65 amino acids long, resulting in a molecular weight of approximately 7,000 daltons.

The hirudin amino acid composition includes a high content of asparagine and glutamine and their carboxylic acid forms ; it lacks arginine, methionine and tryptophan. Furthermore it contains 6 cysteine residues and a COOH-terminal acidic portion that carries a stretch of homology with a thrombin cleavage site in prothrombin, and consequently is important for the binding to the recognition site of thrombin. Replacement of the two N-terminal valyl residues of recombinant hirudin by the polar amino acids Leucine and Threonin results in an increase in the inhibition constant. Thus the hydrophobic nature of the N-terminal residues of hirudin is important for its interaction with thrombin [5].

Recombinant hirudin inhibits both clot-bound and fluid-phase thrombin. A pure and specific antithrombin, r-hirudin has been used successfully in the management of acute coronary syndromes. In fact, r-hirudin can be clinically used for the prevention of early reocclusion after thrombolysis, the prophylaxis of thromboembolic complications, in the case of instable angina pectoris, and for the treatment of DVT and HIT type II.

If a use of hirudin in humans is intended, it has to be highly purified. This purification has to be monitored by a wide range of studies. To obtain evidence for the primary and secondary structure, the amino acid sequence and composition, the peptide map etc. HPLC and HPCE (CZE) have to be used. Besides the desired hirudin, some or more "related products" like conformational variants, degradation products etc. are detected in such investigation.

### Capillary Zone Electrophoresis

Free solution capillary electrophoresis, also called capillary zone electrophoresis (CZE), is the most common mode of capillary electroseparation, because it is performed in a capillary filled only with an electrolyte solution at a selected pH value and ionic strength.

CZE is applicable to a wide range of charged substances including pharmaceuticals, amino acids, peptides and proteins.

The separation in CZE is based on the differences in the electrophoretic mobilities resulting in different velocities of migration of ionic species in the electrophoretic buffer contained in the capillary. The higher the viscosity or the larger the charged molecule, the more the resistance and the slower it moves.

The narrow bore capillaries used in HPCE are generally produced by heating a widebore, fused-silica capillary in a high-temperature oven and mechanically pulling the capillary to obtain the desired capillary diameter [6]. At these high temperatures, the silanols on the capillary become fused (Si-O-Si) and must be regenerated by flushing a brandnew capillary with 1 M sodium hydroxide for about 1 hour to activate the silanols (Si-OH), which may become ionized in the presence of the electrophoretic medium.

The interface between the fused silica tube wall and the electrophoretic buffer consists of three layers: the negatively charged silica surface (at pH > 2), the immobile layer (fixed negative charges are matched by an equal number of solvated cations), and the diffuse layer of cations (and their sphere of hydration) adjacent to the surface of the silica tend to migrate towards the cathode. This migration of cations results in a concomitant migration of fluids through the capillary.

The electrically induced flow of liquid through the capillary is called electroosmotic flow (EOF). It is strongly affected by pH and significantly greater than the electrophoretic mobility of the individual ions and charged molecules resp. in the injected sample. Consequently, both negative and positive charged structures can be separated in the same CZE run, at which cations with the highest charge/mass ratio migrate first, followed by cations with reduced ratios. Finally the anions with the highest charge/mass ratio and thus the greatest electrophoretic mobilities migrate last passing the detector window near the cathodic side resp.

The electroosmotic velocity can be adjusted by controlling the pH (since more silanol groups are ionized, both the zeta potential and the flow increase), the viscosity (as viscosity increases the velocity decreases), the ionic strength (because of its effect on the zeta potential), the voltage (Flow increases proportionally to voltage), and the dielectric constant of the buffer[7].

The key element contributing to the success of a CZE separation is the composition of the buffer. The type of buffer, its pH, ionic strength, and additives as well as viscosity and last but not least the properties of the capillary wall affect the efficiency, selectivity, and resolution of the separation.

### Theoretical Considerations concerning the Protein Separation

Whereas the separation of small peptides (< 10 amino acids) in the CZE mode often is relatively straightforward and well understood it appears that no single strategy is applicable for large peptides and proteins. As might be expected this is due largely to the wide diversity and complexity associated with these biomolecules [8].

One of the main problems associated with protein separations using CE on untreated fused silica capillaries (CZE) is adsorption by the charged sites of proteins on fixed, negatively charged sites (silanol groups) on the capillary wall. The electrostatic adsorption of proteins on the capillary wall can simply overcome by working at extremes of pH. Under these conditions, the silanol groups on the wall surface are either negatively charged or neutral, as can be represented with the equilibrium:

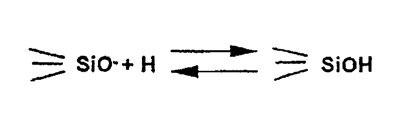

In solution, proteins exhibit acid-base behavior and their net charge is dependent on the pH of the buffer. The net charge is zero at the characteristic isoelectric point (where the number of positive charges is balanced precisely by the number of negative charges). One important point to note is that it is possible to change the charge-to-mass ratio of many charged structures by adjusting the pH of the buffer medium to affect their ionization and hence electrophoretic mobility [9].

Concerning the interaction of proteins with the negatively charged capillary wall at which adsorption is much more critical for larger molecules like proteins because of their high number of binding sites, a "static" approach through permanent modification of the capillary wall by suitablecoatings or a "dynamic" approach using certain buffer additives are suitable tools to reduce or prevent protein adsorption respectively [10].

### Use of Buffer Additives to Optimize Protein Separation

An effective way to achieve better selectivity in CZE is the addition of Ion-Pairing Reagents, e.g. hexanesulfonic acid (HSA). The mechanism by which resolution enhancement occurs is by a hydrophobic pairing between the short alkyl chain of the sulfonate and hydrophobic surfaces on the protein. This results in an increase in negative charge on the protein surface and a corresponding increase in migration time. Yet another mechanism may be at work, in that the HSA decreases the surface positive charge on the proteins and any wall interaction with the negatively charged capillary surface. It is probably a combination of hydrophobic forces and ion pairing at work. Zwitterionic buffer reagents - e.g. aminoethanesulfonic acid - contain both positive and negative ionisable groups, where a secondary amine group provides the positive charge whilst a sulphonic acid group carries the negative charge [11]. Zwitterionic buffers have a number of properties which make these materials eminently suitable as buffers for CZE. They do not contribute to the conductivity and are effective over a wide pH range. Zwitter ions shield the electrostatic attraction as the positively charged ammonium functionality interacts with the negatively charged silanol groups on the capillary surface [12].

While adsorption on the capillary wall is usually not a problem with small peptides, larger peptides and proteins can be irreversibly adsorbed on the capillary wall. This is one of the major problems encountered with FSZE of proteins.

Efforts to reduce adsorption have been aimed at modifying the interfacial double layer at the capillary wall. This can be done by adding small amounts (1 to 5 mmol) of cationic divalent amines e.g. 1,5-diaminopentane to the separation buffer. Divalent amines suppress solute - capillary wall interactions. Note that increasing the amine concentration in the buffer results in longer separations, as the EOF becomes increasingly suppressed.

### Buffer Development

The choice of the optimal run buffer is the most important thing in the CE separation of proteins. The type of buffer, its ionic strength and its pH must be varied for a specific separation problem.

Buffers based on acetate, borate, citrate, phosphate, sulfate or combinations thereof are frequently used in CZE. They are active participants in different systems. Phosphate buffers e.g. may dynamically modify the capillary wall by converting residual silanols on the capillary surface to phosphate complexes that are more easily protonated.

Between pH 2 and 8, the silanol (Si-OH) groups are partially ionized, giving a fixed negative charge to the capillary wall. The negative charges on the wall are matched by an equal number of solvated cations. Under an applied potential these cations migrate toward the cathode imparting a flow to the liquid in the capillary. This electroosmotic flow (EOF) depends on the pH of the buffer and the applied field strength [13]. With untreated fused-silica capillaries the use of high - pH buffers generally produces fast separations because the electroosmotic flow is high. At a low pH peptides and proteins migrate primarilly on the basis of their charge characteristics and the EOF is diminished.

Both electrophoretic mobility and electroosmotic flow decrease with increasing ionic strength. Thus the resulting current should be in the range of 50 µA, but due to Joule heating which leads to temperature gradients and laminar flow not more than 100 µA. Smaller diameter capillaries (25 µm) are more effective concerning heat dissipation, but give reduced sensitivity with UV detection because of the reduced pathlength.

On the other hand enlarging the inner diameter to 75 µm leads to a decrease in resolution. So from a heat dissipating and resolution perspective, the use of a fused silica capillary with an inner diameter of 50 µm is advantegeous.

From an analysis time perspective, capillaries as short as possible should be used, but this is often in contrast to a good resolution. Longer capillaries can produce the separation of more components. But this necessary contributes to band broadening because of longer separation times. So the capillary length needs to be adapted concerning the specific separation, but should not exceed 100 cm in effective length.

Detection in CZE is a significant challenge as a result of the small dimensions of the capillary and the nanoliter sample volumes. An adequate sample concentration is therefore necessary, presuming an ideal injection time of 1 sec., corresponding to a volume of appr. 1 nl. Concerning the fact that higher wavelengths give lower sensitivity and on the other hand most CZE buffers don't absorb at low wavelengths, thus 190 to 200 nm can be used for general purpose detection [14]. A benefit of the air thermostating system, used in the Applied Biosystems CE apparatus is instrumental simplicity and ease of use. Further the system isolates the meandering installed capillary from changes in ambient temperature. An oven temperature of 30°C proposed by the manufacturer of the CE instrument proved to be a desirable condition.

Concerning the separation of r-Hirudin there are two buffer strategies conceivable: (1) working at acidic pH of 3 or (2) working at neutral pH of 7. Working above and below the pl value (which is nearly 4 for r-Hirudin) will change the solute charge and cause the solute to migrate either before or after the EOF.

At a buffer pH of 3 the r-Hirudin possesses a slightly positive charge thus endeavoured comigrating together with the suppressed EOF towards the cathode. In this case, when working with low pH conditions, the capillary wall is protonated while the r-Hirudin molecules are positively charged, thus minimizing electrostatic adsorption on the capillary wall. At pH 3 the EOF is very low and the r-Hirudin is mainly forced moving towards the cathode by it's positive charge.

At neutral pH the pl of the r-Hirudin is less than the pH of the buffer, thus the electrophoretic mobility is towards the anode competing with the electroosmotic mobility which is towards the cathode. At high pH a relatively high EOF is generated and finally the predominating EOF prevents the negatively charged solute (r-Hirudin) from migrating back off the capillary and carries the solute toward the cathode, passing the detector window just before reaching the cathodic side. Both the r-Hirudin and the capillary wall are negatively charged, and the adsorption process is minimized as a result of a charge repulsion effect.

No less than 100 buffers, mainly differing in pH and composition were tested. In connection with this the following chemicals were used:
Buffer salts
   Disodium hydrogen phosphate
   Ammonium sulphate
   Sodium chloride
   Tetramethyl ammoniumsulphate
   Sodium acetate
   Sodium dihydrogen phosphate
   Tris (hydroxymethyl) aminomethane HCI
   Zinc chloride
Buffer acids / bases
   Boric acid
   Formic acid
   Citric acid
   Phosphoric acid
   Sulphuric acid
   Sodium hydroxide
   Acetic acid
   Oxalic acid
Ion-Pairing Reagents
   Hexanesulfonic acid
Zwitterionic salts
   Aminoethanesulfonic acid (Taurin)
   Morpholinoethanesulfonic acid (MES)
Divalent Cationic Amines
   Diaminobutane
   Diaminopentane
   Diaminononane
Zeta Potential Suppressors
   Hydroxypropylmethylcellulose
Ionic Surfactants
   Sodium dodecylsulphate (SDS)
Organic Solvents
   Acetonitrile
Non-Ionic Surfactants
   Polyethylene glycol

Finally the ionic strength of the optimized buffer composition and the length of the capillary were evaluated.

### Results

In order to develop a method for the separation of recombinant hirudin and its related substances, no less than 100 buffers were tested by using free solution capillary electrophoresis (FSCE) also known as capillary zone electrophoresis (CZE).
As an example the results achieved with two different buffer systems can be seen in Fig. 1 and 2. Figure 3 shows the electropherogram achieved with the optimized buffer system. In total, nine related substances are baseline separated from the main peak.

Despite the resolution achievable the use of a rather long capillary is necessary. In consequence the competition of the r-hirudin molecules and the opposite directed EOF leads to a long analysis time.

It is well established that optimal conditions for the CE separation are extremely protein-specific [15].

The capillary zone electrophoretic separation of r-Hirudin is possible in an uncoated capillary using a complex buffer system containing an Ion-Pairing reagent (sulfonic acid), a Zwitterionic buffer reagent and a divalent amine as buffer additives. Sulfonic acids improve resolution through ion-pairing, zwitterionic substances improve resolution and peak shape and amines reduce adsorption and tailing.

One embodiment of the present invention therefore is a buffer system for the CZE analysis of hirudin comprising aminoethanesulfonic acid, hexanesulfonic acid, sodium phosphate, borate, tetramethylammonium sulfate and diaminopentane, adjusted to pH 6 - 8, preferably such a buffer system comprising 30 - 50 mM Aminoethanesulfonic acid, 20 - 30 mM Hexanesulfonic acid, 20 - 80 mM Sodium phosphate,
> 0 - 20 mM Borate, > 0 - 10 mM Tetramethylammonium sulfate, > 0 - 5 mM Diaminopentane, adjusted to pH 6 - 8, and in particular such a buffer system comprising
40 mM Aminoethanesulfonic acid, 25 mM Hexanesulfonic acid, 50 mM Sodium phosphate, 10 mM Borate, 2 mM Tetramethylammonium sulfate, 1 mM Diaminopentane, adjusted to pH 7.

A further embodiment of the invention is a process for the preparation of a buffer system as described above comprising the steps:
(a) mixing the components of the buffer system in a suitable manner; and
(b) adjusting the pH in a suitable manner.

Another embodiment of the invention is the use of a buffer system as described above for the capillary zone electrophoretic analysis of hirudin, preferably, wherein the hirudin is a biochemically purified, naturally occuring hirudin or a recombinantly produced hirudin, in particular [Leu¹, Thr²]-63-Desulfato-hirudin.

A further embodiment of the invention is a process for the separation of hirudin and related products in a capillary zone electrophoretic analysis wherein a buffer system as described above is used.

The invention is now described in more detail by the examples without being limited thereto.

### Example 1: Preparation of the CZE buffer

Chemicals: Concerning the optimized buffer composition the following chemicals were purchased from Fluka: 1,5-Diaminopentane, 1-Hexanesulfonic acid Sodium salt Monohydrate and Tetramethylammoniumsulfate. 2-Aminoethanesulfonic acid and disodium hydrogenphosphate were purchased from Merck. Boric acid and Sulfuric acid were from Riedel-de-Haen.

All solutions were made with Milli-Q (Millipore) water and filtered through an 0,2 micron-filter before use.

The stock solutions were as follow:
250 mM Disodiumhydrogen phosphate, 500 mM Boric acid, 200 mM Tetramethylammoniumsulfate and Sulphuric acid.
1 g 2-Aminoethanesulfonic acid and 1,02 g Hexanesulfonic acid Sodium salt Monohydrate were dissolved in 140 ml. 40 ml Disodiumhydrogen phosphate (250 mM), 4 ml Boric acid (500 mM), 2 ml Tetramethylammoniumsulfate (200 mM) and 24 µl 1,5-Diaminopentane were then added.

The buffer solution was adjusted to pH 7 with 1 M Sulphuric acid. Finally the volume was completed to 200 ml, which results in the following buffer system:
40 mM Aminoethanesulfonic acid
25 mM Hexanesulfonic acid
50 mM Sodium phosphate
10 mM Borate
2 mM Tetramethylammonium sulfate
1 mM Diaminopentane
   adjusted to pH 7

### Example 2: CE separation conditions

CE instrumentation: The CE measurements were carried out on an Applied Biosystems 270A-HT capillary electrophoresis apparatus, equipped with a monochromatic UV detector. Data collection was recorded via a Perkin Elmer 900 series interface. All peak information (migration time, peak area and height, area percent) was obtained through the Nelson PC-integrator software version 5.1.

Prior to use a brandnew capillary was equilibrated with 1 M Sodium hydroxide for 2 h, with 0.1 M sodium hydroxide for 20 min., with Milli-Q water for 15 min., and finally with the separation buffer for 10 minutes.

The polyimide-coated, fused silica capillary (Supelco, Bellefonte, PA) was 50µm ID and 101 cm in length (80 cm effective length, respective to the detector). The normal polarity technique was used, in which the sample is applied at the anodic side. The capillary oven temperature was maintained at 30°C and all runs were performed at 20 kV, corresponding to a current of 46 to 50 µA.

Detection was measured by absorbance at 200 nm. The samples were loaded for 1 s by vacuum injection, using a 5-inch vacuum (16,9 kPa), corresponding to a sample volume of about 1 nl [14].

Between individual runs, the capillary was rinsed with Milli-Q water for 2 min., with 1 M sodium hydroxide for 2 min., with 0.1 M sodium hydroxide for 2 min., and finally with the separation buffer for 6 minutes to prepare the capillary for the next separation.

After around 30 HPCE runs the capillary needs to be conditioned as follows due to the decline in separation efficiency:
30 min. with 1 M NaOH, 10 min. with 0.1 M NaOH, and finally 10 min. with Milli-Q water.

Concerning the storage of the capillary only used for this specific separation, flushing the capillary with water for 10 min. and then flushing from an empty vial for 5 minutes is the standard practice. The 2nd rinse fills the capillary with air and prepares the capillary for a dry storage without plugging.

### Figure legends

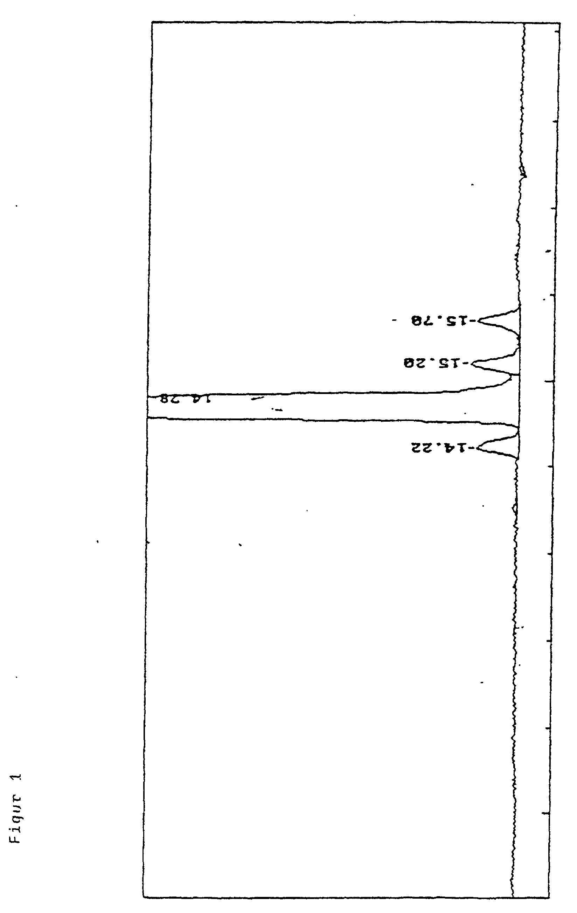

Fig. 1: Example of an CZE analysis of [Leu¹, Thr²]-63-Desulfato-hirudin. Buffer 60mM Citrate, 20mM Tris HCI, pH 3 Capillary: 72cm, 50pm; Conditions: 190nm, 30°C, 25kV, 35µA

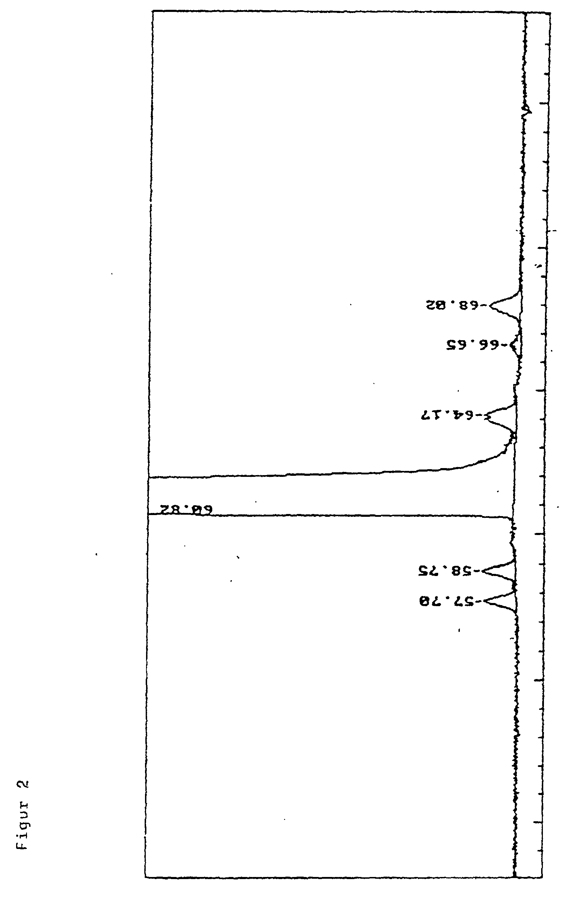

Fig. 2: Example of an CZE analysis of [Leu¹, Thr²]-63-Desulfato-hirudin. Buffer: 100mM Phosphate, pH 3.1; Capilary: 72cm, 50µm; Conditions: 190nm, 30°C, 15kV, 65µA

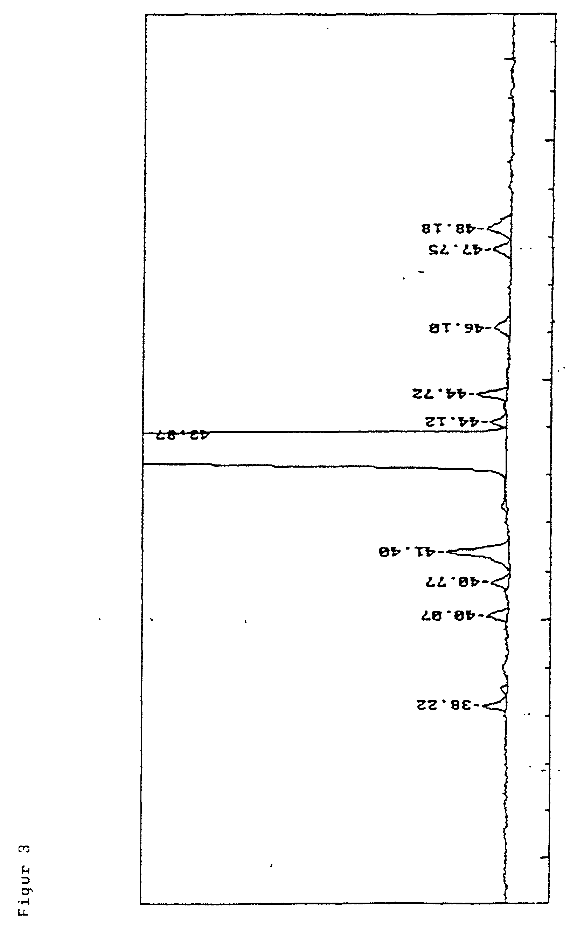

Fig. 3: Example of an CZE analysis of [Leu¹, Thr²]-63-Desulfato-hirudin. Buffer: 50mM Phosphate, 40mM Aminoethanesulfonic acid, 25mM Hexanesulfonic acid, 10mM borate, 2mM Tetramethylammonium sulfate, 1mM Diaminopentane, pH 7; Capillary: 101cm, 50µm; Conditions: 200nm, 30°C, 20kV, 47µA

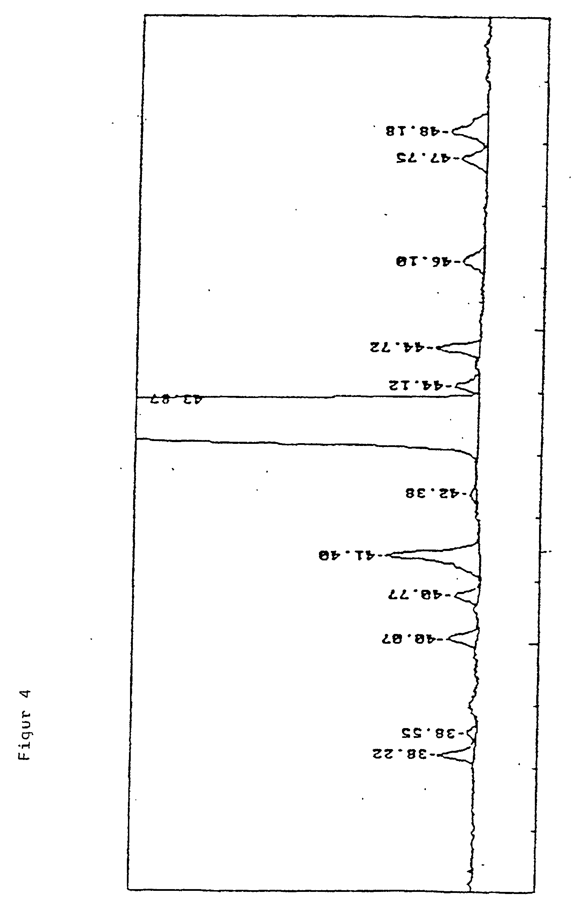

Fig. 4: Integration result of the CZE analysis of [Leu¹, Thr²]-63-Desulfato-hirudin as shown in Fig. 3.

**Table 1:**

| | | | Run Time: | 62.00 |
|---|---|---|---|---|
| Pk No. | Ret Time | Peak Area | Area % | Peak Ht. |
| 1 | 38.217 | 5689 | 0.2204 | 657 |
| 2 | 38.550 | 1488 | 0.0577 | 166 |
| 3 | 40.067 | 5168 | 0.2002 | 542 |
| 4 | 40.767 | 4160 | 0.1612 | 441 |
| 5 | 41.400 | 21822 | 0.8455 | 1717 |
| 6 | 42.383 | 1569 | 0.0608 | 127 |
| 7 | 43.867 | 2507941 | 97.1717 | 110399 |
| 8 | 44.117 | 3988 | 0.1545 | 424 |
| 9 | 44.717 | 6973 | 0.2702 | 801 |
| 10 | 46.100 | 5558 | 0.2153 | 405 |
| 11 | 47.750 | 5880 | 0.2278 | 479 |
| 12 | 48.183 | 10702 | 0.4147 | 679 |
| Total Area: | | 2580937 | | |

### Abbreviations

DVT, deep vein thrombosis ; HIT, heparin induced thrombocytopenia ; UF, ultra filtration ; RP-HPLC, reversed phase - high performance liquid chromatography ; HPCE, high performance capillary electrophoresis ; CZE, capillary zone electrophoresis ; FSZE, fused silica zone electrophoresis ; FSCE, free solution capillary electrophoresis ; CE, capillary electrophoresis ; EOF, electroendosmotic flow ; UV, ultra-violet; ID, inner diameter ; HT, high throughput.

### References

[1] Walenga, J.M., Pifarre, R., Hoppenstaedt, D.A., Fareed, J. Development of Recombinant Hirudin as a Therapeutic Anticoagulant and Antithrombotic Agent: Some Objective Considerations
   Seminars in Thrombosis and Hemostasis, Volume 15, No. 3, 316-333 (1989)
[2] Johnson, P.H., Sze, P., Winant, R., Payne, P.W., and Lazar, J.B. Biochemistry and Genetic Engineering of Hirudin
   Seminars in Thrombosis and Hemostasis, Volume 15, No. 3, 302-315 (1989)
[3] Courtney, M., Loison, G., Lemoine, Y., Riehl-Bellon, N., Degryse, E., Brown, S.W., Cazenave, J.P., Defreyn, G., Delebassee, D., Bernat, A., Maffrand, J.P., and Roitsch, C.
   Production and Evaluation of Recombinant Hirudin Seminars in Thrombosis and Hemostasis, Volume 15, No. 3, 288-292 (1989)
[4] Haruyama, H., and Wuethrich, K.
   Conformation of Recombinant Desulfatohirudin in Aqueous Solution Determined by Nuclear Magnetic Resonance Biochemistry, Volume 28, 4301-4312 (1989)
[5] Wallace, A., Dennis, S., Hofsteenge, J., and Stone, S.R. Contribution of the N-Terminal Region of Hirudin to Its Interaction with Thrombin
   Biochemistry, Volume 28, 10079-10084 (1989)
[6] Altria, K.D., Bryant, S.M., Clark, B.J., Kelly, M.A. The Care and Maintenance of CE Capillaries
   LC-GC International, 157-162 (1997)
[7] Li, S.F.Y. Capillary Electrophoresis
   Journal of Chromatography Library, Volume 52, 1992
[8] Schwartz, H., and Pritchett, T.
   Separation of Proteins and Peptides by Capillary Electrophoresis: Application to Analytical Biotechnology
   Beckman Instruments, Inc. (1994)
[9] Schwartz, H.E., Palmieri, R.H., Nolan, J.A., Brown, R.
   Introduction to Capillary Electrophoresis of Proteins and Peptides
   Beckman Instruments, Inc. (1993)
[10] Waetzig, H., and Dette, C.
   Capillary electrophoresis (CE) - a review Strategies for method development and applications related to pharmaceutical and biological sciences Pharmazie, Volume 49, Issue 2/3, 83-96 (1994)
[11] Good, N.E., and Izawa, S.
   Hydrogen Ion Buffers
   Methods in Enzymology, Volume XXIV, p. 53-68 (1972)
[12] Bushey, M.M., and Jorgenson, J.W.
   Capillary Electrophoresis of proteins in buffers containing high concentrations of zwitterionic salts
   Journal of Chromatography, Volume 480, 301-310 (1989)
[13] Saunders, D.
   CE Method Development
   LC Resources, Walnut Creek, CA (1994)
[14] Hermentin, P., Doenges, R., Witzel, R., Hokke, C.H., Vliegenthart, J.P.K., Conradt, H.S., Nimtz, M., and Brazel D.
   A Strategy for the Mapping of N-Glycans by High-Performance Capillary Electrophoresis
   Analytical Biochemistry, Volume 221, 29-41 (1994)
[15] Oda, R.P., and Landers, J.P.
   Effect of cationic buffer additives on the capillary electrophoretic separation of serum transferrin from different species Electrophoresis, Volume 17, 431-437 (1996)

## Claims

1. Buffer system for the capillary zone electrophoretic (CZE) analysis of hirudin comprising aminoethanesulfonic acid, hexanesulfonic acid, sodium phosphate, borate, tetramethylammonium sulfate and diaminopentane, adjusted to pH 6 - 8.

2. Buffer system according to claim 1 comprising
30 - 50 mM Aminoethanesulfonic acid
20 - 30 mM Hexanesulfonic acid
20 - 80 mM Sodium phosphate
> 0 - 20 mM Borate
> 0 - 10 mM Tetramethylammonium sulfate
> 0 - 5 mM Diaminopentane
adjusted to pH 6 - 8.

3. Buffer system according to claim 2 comprising
40 mM Aminoethanesulfonic acid
25 mM Hexanesulfonic acid
50 mM Sodium phosphate
10 mM Borate
2 mM Tetramethylammonium sulfate
1 mM Diaminopentane
adjusted to pH 7

4. Process for the preparation of a buffer system according to any of claims 1 to 3 comprising the steps
(c) mixing the components of the buffer system in a suitable manner; and
(d) adjusting the pH in a suitable manner.

5. Use of a buffer system according to any of claims 1 to 3 for the capillary zone electrophoretic analysis of hirudin.

6. Use according to claim 5, wherein the hirudin is a biochemically purified, naturally occuring hirudin.

7. Use according to claim 5, wherein the hirudin is a recombinantly produced hirudin.

8. Use according to claim 7, wherein the recombinantly produced hirudin is [Leu¹, Thr²]-63-Desulfato-hirudin.

9. Process for the separation of hirudin and related products in a capillary zone electrophoretic analysis wherein a buffer system according to any of claims 1 to 3 is used.

## Patentansprüche

1. Puffersystem für die kapillarzonenelektrophoretische (CZE)-Analyse von Hirudin, umfassend Aminoethansulfonsäure, Hexansulfonsäure, Natriumphosphat, Borat, Tetramethylammoniumsulfat und Diaminopentan, eingestellt auf pH 6-8.

2. Puffersystem nach Anspruch 1, umfassend
30 - 50 mM Aminoethansulfonsäure
20 - 30 mM Hexansulfonsäure
20 - 80 mM Natriumphosphat
> 0 - 20 mM Borat
> 0 - 10 mM Tetramethylammoniumsulfat
> 0 - 5 mM Diaminopentan
eingestellt auf pH 6-8.

3. Puffersystem nach Anspruch 2, umfassend
40 mM Aminoethansulfonsäure
25 mM Hexansulfonsäure
50 mM Natriumphosphat
10 mM Borat
2 mM Tetramethylammoniumsulfat
1 mM Diaminopentan
eingestellt auf pH 7

4. Verfahren zur Herstellung eines Puffersystems nach einem der Ansprüche 1 bis 3, das die Schritte
(c) Mischen der Bestandteile des Puffersystems in geeigneter Weise; und
(d) Einstellen des pH-Werts in geeigneter Weise umfaßt.

5. Verwendung eines Puffersystems nach einem der Ansprüche 1 bis 3 zur kapillarzonenelektrophoretischen Analyse von Hirudin.

6. Verwendung nach Anspruch 5, wobei es sich bei dem Hirudin um ein biochemisch gereinigtes, natürlich vorkommendes Hirudin handelt.

7. Verwendung nach Anspruch 5, wobei es sich bei dem Hirudin um ein rekombinant hergestelltes Hirudin handelt.

8. Verwendung nach Anspruch 7, wobei das rekombinant hergestellte Hirudin [Leu¹, Thr²]-63-Desulfatohirudin ist.

9. Verfahren zur Trennung von Hirudin und verwandten Produkten in einer kapillarzonenelektrophoretischen Analyse, wobei ein Puffersystem nach einem der Ansprüche 1 bis 3 verwendet wird.

## Revendications

1. Système tampon pour l'analyse par électrophorèse capillaire de zone (CZE) de l'hirudine, comprenant l'acide aminoéthanesulfonique, l'acide hexanesulfonique, le phosphate de sodium, le borate, le sulfate de tétraméthylammonium et le diaminopentane, ajusté à un pH de 6 à 8.

2. Système tampon selon la revendication 1, comprenant
de 30 à 50 mM d'acide aminoéthanesulfonique
de 20 à 30 mM d'acide hexanesulfonique
de 20 à 80 mM de phosphate de sodium
de > 0 à 20 mM de borate
de > 0 à 10 mM de sulfate de tétraméthylammonium
de > 0 à 5 mM de diaminopentane
ajusté à un pH de 6 à 8.

3. Système tampon selon la revendication 2, comprenant
40 mM d'acide aminoéthanesulfonique
25 mM d'acide hexanesulfonique
50 mM de phosphate de sodium
10 mM de borate
2 mM de sulfate de tétraméthylammonium
1 mM de diaminopentane
ajusté à un pH de 7.

4. Procédé de préparation d'un système tampon selon l'une quelconque des revendications 1 à 3, comprenant les étapes consistant à
(c) mélanger les composants du système tampon de façon appropriée ; et
(d) ajuster le pH de façon appropriée.

5. Utilisation d'un système tampon selon l'une quelconque des revendications 1 à 3, pour l'analyse par électrophorèse capillaire de zone de l'hirudine.

6. Utilisation selon la revendication 5, dans laquelle l'hirudine est une hirudine présente dans la nature, biochimiquement purifiée.

7. Utilisation selon la revendication 5, dans laquelle l'hirudine est une hirudine produite par voie recombinante.

8. Utilisation selon la revendication 7, dans laquelle l'hirudine produite par voie recombinante est la [Leu¹,Thr²]-63-désulfato-hirudine.

9. Procédé de séparation de l'hirudine et de produits apparentés dans une analyse par électrophorèse capillaire de zone, dans lequel un système tampon selon l'une quelconque des revendications 1 à 3 est utilisé.
